# FASCICULE DE BREVET EUROPEEN

(11) **EP 1 446 233 B1**
(45) Date de publication et mention de la délivrance du brevet: **25.01.2006**
(21) Numéro de dépôt: 02788054.1
(22) Date de dépôt: 14.11.2002
(51) Int. Cl.: B05B 11/02, A61M 5/28

(54) **DISPOSITIF DE DISTRIBUTION DE PRODUIT FLUIDE**
ABGABEVORRICHTUNG FÜR MEDIEN
FLUID PRODUCT DISPENSING DEVICE

(30) Priorité: 15.11.2001 FR 0114794
(43) Date de publication de la demande: 18.08.2004
(73) Titulaire: Valois SAS, 27110 Le Neubourg (FR)
(72) Inventeur: HELDT, Frédéric, F-27400 Louviers (FR); STRADELLA, Fabio, I-16032 Camogli (IT); STRADELLA, Giuseppe, I-16032 Camogli (IT)
(74) Mandataire: CAPRI
(86) Numéro de dépôt international: PCT/FR2002/003900
(87) Numéro de publication internationale: WO 2003/041872

(56) Documents cités:
- EP-A- 0 546 607
- WO-A-01/30423
- DE-A- 1 491 663
- DE-A- 19 837 127
- US-A- 3 387 609
- US-A- 4 623 337

## Description

La présente invention concerne un dispositif de distribution de produit fluide, et plus particulièrement un tel dispositif destiné à distribuer un faible nombre de doses, tel qu'un monodose ou un bidose.

Les dispositifs de distribution de produit fluide qui ne contiennent que peu de doses doivent pouvoir être réalisés à faible coût, tout en garantissant un stockage et une distribution sûrs et fiables du produit.

Le document DE-A-19837127 montre un dispositif de distribution de produit fluide selon le préambule de la revendication 1.

Les documents WO 95/27568 et EP-0 546 607 divulguent deux dispositifs de ce type. Le dispositif du document WO 95/27568 comporte un réservoir pourvu d'une seule ouverture qui est obturée par un bouchon perçable. L'aiguille de percement est solidaire du corps et lors de l'actionnement, ce corps avec l'aiguille se déplace par rapport au réservoir en direction du bouchon pour le percer, le bouchon étant ensuite entraîné en déplacement à l'intérieur du réservoir, et se transformant donc en piston, pour distribuer le contenu du réservoir à travers ladite aiguille. Le dispositif du document EP-0 546 607 est similaire, à savoir qu'il comporte également une aiguille de percement solidaire du corps. La différence par rapport au document précédent est que dans ce dispositif, c'est le réservoir qui est déplacé par l'utilisateur par rapport à ladite tête, de sorte que lors de l'actionnement, le bouchon est d'abord percé, puis ensuite déplacé à l'intérieur du réservoir, en se transformant en piston, pour distribuer le produit à travers l'aiguille.

Dans ces deux exemples susmentionnés, le réservoir est obturé par un bouchon qui se transforme en piston après percement dudit bouchon. D'autre part, le réservoir est toujours monté mobile par rapport au corps pour réaliser le déplacement du piston à l'intérieur du réservoir lors de la distnbution. Ceci implique un montage et un remplissage du dispositif relativement compliqué.

Les documents DE-1 491 663, US-3 387 609 et WO 01/30423 divulguent des seringues. Dans ce type de distributeurs, toute précompression du produit est néfaste et non souhaitable pour l'utilisateur. Il en est de même pour le document US-4 623 337 qui divulgue un distributeur de gouttes pour les yeux.

La présente invention a pour but de fournir un dispositif de distribution de produit fluide qui ne comporte pas les inconvénients susmentionnés.

La présente invention a pour but de fournir un dispositif de distribution de produit fluide différent qui soit simple et peu coûteux à fabriquer et à assembler.

La présente invention a également pour but de fournir un tel dispositif de distribution de produit fluide dans lequel le remplissage du réservoir avec le produit fluide est simplifié.

La présente invention a aussi pour but de fournir un tel dispositif qui garantit une parfaite pulvérisation du produit et la distribution de la totalité de la dose de produit.

La présente invention a donc pour objet un dispositif de distribution de produit fluide, comportant un corps pourvu d'un orifice de distribution, un réservoir contenant au moins une dose de produit fluide étant disposé fixement dans ledit corps, ledit réservoir étant obturé de manière étanche par deux bouchons, un premier bouchon et un second bouchon, le produit fluide étant disposé dans une chambre de produit située entre lesdits premier et second bouchons, ledit dispositif comportant des moyens d'ouverture dudit premier bouchon, lesdits moyens d'ouverture étant disposés fixement dans ledit corps, des moyens d'actionnement étant reliés audit second bouchon pour le déplacer dans ledit réservoir, l'actionnement dudit second bouchon créant une pression dans la chambre de produit, ladite pression déplaçant et/ou déformant ledit premier bouchon vers lesdits moyens d'ouverture, de telle sorte que ledit premier bouchon est d'abord ouvert, puis du produit est expulsé à travers ledit premier bouchon ouvert vers ledit orifice de distribution, un profil de pulvérisation étant prévu en aval dudit orifice de distribution, le dispositif comportant des moyens de précompression pour créer une précompression dans le réservoir avant l'ouverture dudit premier bouchon par lesdits moyens d'ouverture, pour améliorer la pulvérisation du produit et assurer la distribution de la totalité de la dose de produit.

Avantageusement, lesdits moyens d'ouverture du premier bouchon comportent une aiguille creuse adaptée à percer ledit premier bouchon et à transférer le produit fluide vers l'orifice de distribution.

Selon une première variante de réalisation, ledit premier bouchon est disposé à l'intérieur dudit réservoir et est déplaçable vers lesdits moyens d'ouverture lors de l'actionnement.

Selon une seconde variante de réalisation, ledit premier bouchon est fixé audit réservoir par une partie fixe et comporte une partie déformable, disposée à l'intérieur dudit réservoir, et se déformant vers lesdits moyens d'ouverture lors de l'actionnement.

Avantageusement, ladite partie fixe du premier bouchon est fixée à l'extérieur du réservoir.

Selon un premier mode de réalisation de l'invention, ledit réservoir contient une dose unique de produit fluide distribuée lors d'un seul actionnement du dispositif.

Selon un second mode de réalisation de l'invention, ledit réservoir contient au moins deux doses de produit fluide distribuées lors d'actionnements successifs des dispositifs, lesdits moyens d'actionnement comportant des moyens de fractionnement de doses.

Avantageusement, lesdits moyens de précompression sont formés sur le premier bouchon et/ ou sur les moyens d'ouverture et/ou sur le réservoir.

Avantageusement, lesdits moyens de précompression comportent des moyens de résistance nécessitant une force d'actionnement supérieure à un seuil prédéterminé pour ouvrir ledit premier bouchon.

Avantageusement, lesdits moyens de résistance comprennent une butée coopérant avec ledit premier bouchon avant son ouverture, ledit premier bouchon devant être déformé pour surmonter ladite butée.

D'autres caractéristiques et avantages de la présente invention apparaîtront plus clairement au cours de la description détaillée suivante de deux variantes de réalisation de celle-ci, faite en référence aux dessins joints, donnés à titre d'exemples non limitatifs, et sur lesquels,
- la figure 1 est une vue schématique en section transversale d'un dispositif de distribution de produit fluide selon un mode de réalisation particulier de la présente invention, avant actionnement,
- les figures 2 et 3 sont des vues schématiques agrandies d'un réservoir selon une première variante de réalisation de la présente invention, respectivement avant et après actionnement,
- les figures 4 et 5 sont des vues similaires à celles des figures 2 et 3, représentant une seconde variante de réalisation de la présente invention, respectivement avant et après actionnement du dispositif, et
- les figures 6, 7, 8 et 9 montrent différentes variantes de réalisation de la présente invention.

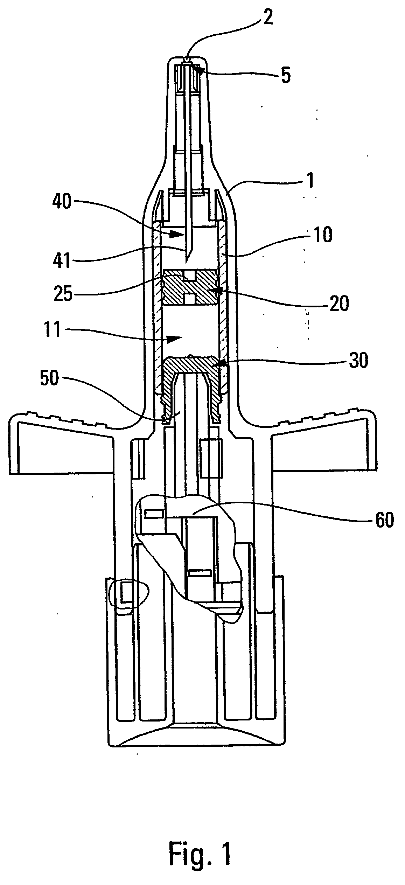

En référence à la figure 1, qui représente un dispositif de distribution du type bidose, c'est à dire contenant deux doses de produit dans le réservoir, le dispositif comporte un corps 1 qui incorpore l'orifice de distribution 2 et qui contient un réservoir 10. Ce réservoir 10 est disposé fixement par rapport audit corps 1. Le réservoir est ouvert des deux côtés et donc obturé de manière étanche par un premier bouchon 20 disposé d'un côté du réservoir 10 et par un second bouchon 30 disposé de l'autre côté dudit réservoir 10. Une chambre de produit 11 est donc définie entre lesdits bouchons 20 et 30, cette chambre de produit 11 contenant le produit fluide à distribuer.

Des moyens d'ouverture 40 du premier bouchon 20 sont prévus, qui sont avantageusement réalisés sous le forme d'une aiguille de percement 40. Cette aiguille de percement 40 est de préférence reliée directement à l'orifice de distribution 2 et formée avec une pointe de percement 41 adaptée à venir percer le bouchon 20, de préférence dans une partie d'ouverture 25 de celui-ci. L'aiguille 40 est de préférence creuse, de sorte qu'une fois le bouchon 20 percé, le produit fluide s'écoule à l'intérieur de l'aiguille 40 en direction de l'orifice de distribution 2. Selon l'invention, un profil de pulvérisation 5 est prévu en aval de l'orifice de distribution 2, afin de distribuer le produit sous forme de spray finement pulvérisé.

Le second bouchon 30 est relié à un dispositif d'actionnement 50, qui peut être un dispositif d'actionnement manuel sur lequel l'utilisateur appuie pour distribuer le produit. Ce bouchon 30 est monté de manière déplaçable à l'intérieur du réservoir 10, de sorte que lorsque l'utilisateur appuie sur le dispositif d'actionnement 50, celui-ci exerce une force axiale sur le second bouchon 30. Cette force axiale crée une pression à l'intérieur de la chambre de produit 11, qui, le produit étant incompressible, est transmise au premier bouchon 20 et déplace celui-ci en direction de l'aiguille 40. Ainsi, le premier bouchon 20 est d'abord percé par l'aiguille 40, puis sous l'effet de la force axiale exercée par l'utilisateur, le produit contenu dans la chambre de produit 11 est distribué à travers ladite aiguille 40 en direction de l'orifice de distribution 2.

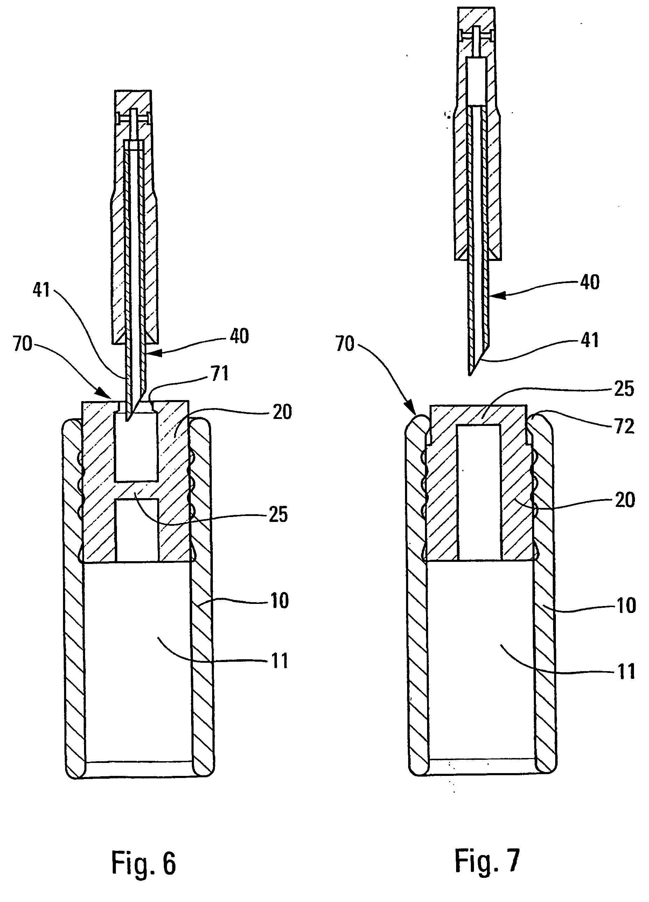

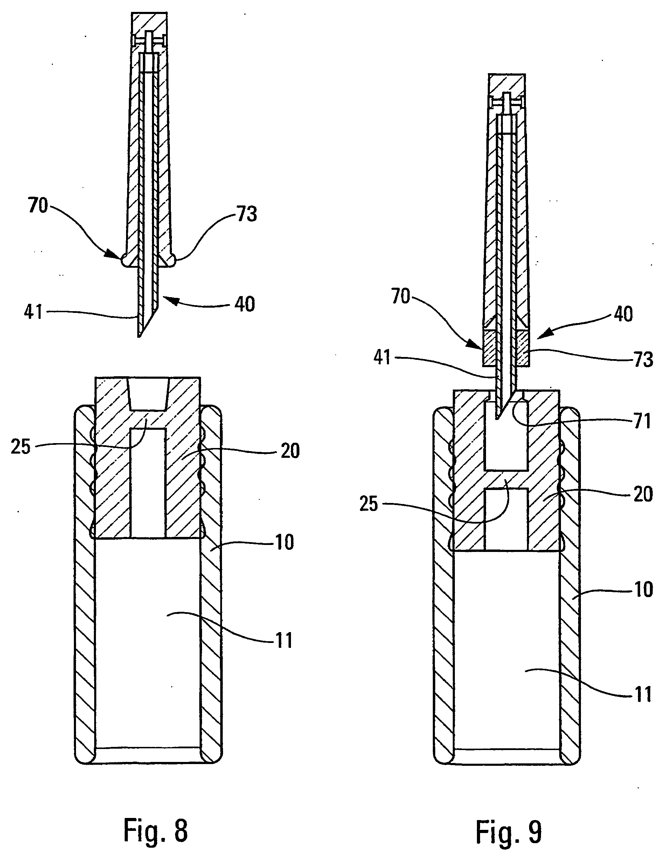

Selon l'invention, des moyens de précompression 70 sont prévus pour créer une précompression avant ouverture du premier bouchon 20. Ceci favorise une bonne pulvérisation du produit. Par ailleurs, ceci fournit également une accumulation d'énergie dans la main de l'utilisateur lors de l'actionnement. Ce phénomène favorise aussi une bonne pulvérisation du produit, et garantit la distribution totale de chaque dose de produit, ce qui n'est pas toujours le cas dans les dispositifs ne prévoyant pas d'accumulation d'énergie dans la main de l'utilisateur et/ou de précompression du produit avant distribution. Les moyens de précompression 70 peuvent être formés sur le premier bouchon 20, comme représenté sur la figure 6, sur le réservoir 10, comme représenté sur la figure 7, ou sur les moyens d'ouverture 40, comme représenté sur les figures 8 et 9. Bien entendu, ces moyens peuvent être combinés entre eux, comme notamment visible sur la figure 9. Ils peuvent comporter des moyens ou zones de résistance 71, 72, 73, formant avantageusement butée et obligeant le bouchon 20 à se déformer pour pouvoir les surmonter. Ceci permet de prédéterminer avantageusement la force seuil nécessaire pour actionner le dispositif, alors que le frottement du bouchon dans le réservoir est un paramètre difficilement maîtrisable.

Le dispositif de la présente invention fournit donc un corps 1 contenant un réservoir 10, le réservoir 10 étant fixé à l'intérieur du corps 1, le corps 1 incorporant également les moyens d'ouverture 40 du premier bouchon 20, en l'occurrence l'aiguille de percement 40, cette aiguille étant donc également fixe par rapport audit réservoir 10. Seuls les bouchons 20 et 30 sont mobiles à l'intérieur du réservoir 10. Le bouchon 20 est percé lors de l'actionnement, et c'est le bouchon 30 qui agit en tant que piston pour distribuer le produit.

Un avantage particulier de cette mise en oeuvre, outre le fait de simplifier la mise en place du réservoir dans le dispositif puisque celui-ci est fixé dans le corps 1 et ne doit pas être déplaçable par rapport à celui-ci lors de l'actionnement, est que le remplissage du réservoir est facilité. En effet, le réservoir 10 de l'invention peut être assemblé dans le corps 1, puis le premier bouchon 20 peut être introduit dans celui-ci. Enfin, le produit est rempli de manière très simple dans le réservoir en amont dudit bouchon 20, et le réservoir est finalement bouché par le second bouchon 30, de manière étanche. Le système d'actionnement 50 peut alors être assemblé sur le corps 1, ceci pouvant être réalisé à tout moment, puisque le réservoir est obturé de manière étanche dès la mise en place du second bouchon 30. Ce type de remplissage n'est pas possible avec les dispositifs antérieurs dans lesquels le réservoir n'est obturé que pour un seul bouchon qui se transforme, après percement, en piston.

L'exemple représenté sur la figure 1 est un bidose. Avantageusement, le système d'actionnement 50 comporte des moyens de fractionnement de doses 60, qui dans cet exemple sont réalisés par une butée 60 qui limite la course du piston 30 (second bouchon) lors du premier actionnement. Le système d'actionnement 50 doit ensuite être déplacé par rapport à cette butée 60, par exemple tourné autour de l'axe central du dispositif, pour pouvoir distribuer la seconde dose. Bien entendu, la présente invention s'applique également aux dispositifs monodoses (appelés aussi unidoses), c'est à dire ne contenant qu'une seule dose de produit distribuée en un seul actionnement. On peut également envisager de prévoir plus de deux doses, par exemple trois ou quatre. Par ailleurs, la figure 1 montre un système d'actionnement 50 sur lequel l'utilisateur exerce une force axiale pour distribuer le produit. Il est possible en variante de prévoir un système d'actionnement latéral, dans lequel l'utilisateur exerce sa force d'actionnement dans une direction différente de la direction de déplacement du piston (second bouchon) lors de la distribution.

La figure 1 représente des moyens d'ouverture 40 réalisés sous la forme d'une aiguille creuse, mais ces moyens d'ouverture 40 pourraient être réalisés de manière alternative, par exemple sous la forme d'un tube creux qui vient déplacer un élément d'obturation coincé dans la partie d'ouverture 25 du premier bouchon 20, par exemple une bille ou similaire. Dans ce cas, cet élément d'obturation est déplacé par les moyens d'ouverture 40 lors de l'actionnement du dispositif et le produit est ensuite distribué à travers ledit tube creux. Ce mode de réalisation n'est pas représenté sur les dessins, mais il pourrait très bien être appliqué à la présente invention. D'autres variantes de moyens d'ouverture sont aussi envisageables.

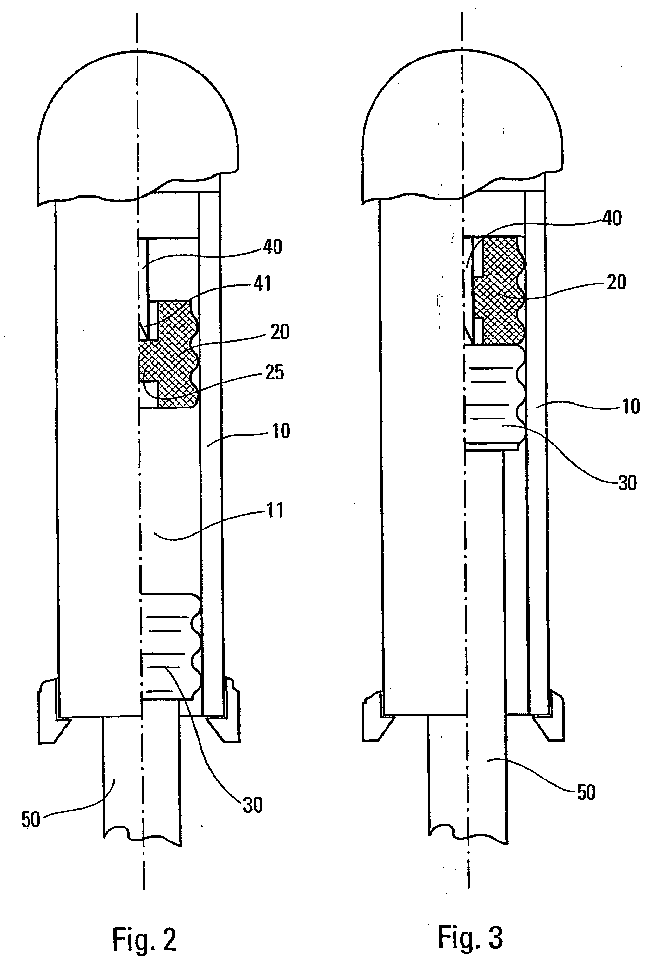

Les figures 2 et 3 montrent des vues agrandies du réservoir, selon une première variante similaire à celle représentée sur la figure 1. Dans cette première variante, le premier bouchon 20 est disposé à l'intérieur du réservoir 10 et coulisse sur la paroi latérale interne dudit réservoir 10 lorsque l'utilisateur actionne le système d'actionnement 50 pour distribuer le produit. Dans cette variante, c'est donc la totalité du premier bouchon 20 qui se déplace par rapport au réservoir, pour être ouvert, notamment percé, par le dispositif d'ouverture 40.

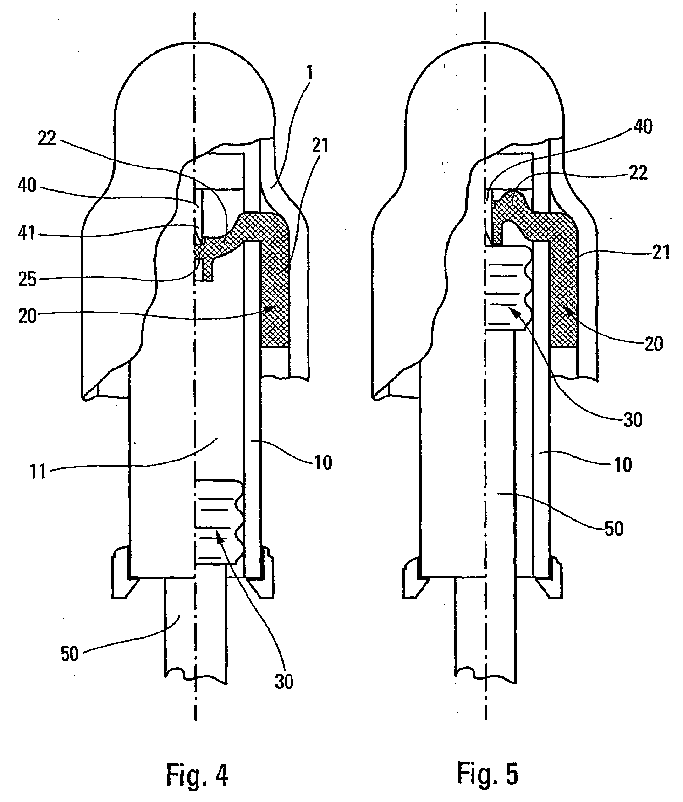

En variante, les figures 4 et 5 montrent un premier bouchon 20 qui n'est pas déplaçable mais déformable par rapport au réservoir 10. A cet effet, ce premier bouchon 20 comporte une partie fixe 21, qui est fixée au réservoir 10, et une partie déformable 22 qui est adaptée à se déformer en direction des moyens d'ouverture 40, lors de l'actionnement du dispositif. Avantageusement, la partie fixe 21 peut être fixée à l'extérieur du réservoir 10 alors que la partie déformable, disposée à l'intérieur du réservoir 10, incorpore la partie d'ouverture 25, qui vient coopérer avec les moyens d'ouverture 40, en l'occurrence la pointe 41 de l'aiguille 40. Comme visible sur la figure 4, cette partie déformable 22 du premier bouchon 20 peut comporter une courbure dirigée vers le bas sur la figure 4, cette courbure provoquant une résistance supérieure à la déformation de cette partie déformable 22 lors de l'actionnement. Ceci favorise aussi la précompression et/ou l'accumulation d'énergie dans la main de l'utilisateur. Il est aussi envisageable de prévoir un premier bouchon à la fois déplaçable et déformable.

La présente invention a été décrite en référence à plusieurs variantes de réalisation, mais il est clair qu'elle n'est pas limitée à ces variantes. Au contraire, un homme du métier peut y apporter toutes modifications sans sortir du cadre de la présente invention tel que défini par les revendications annexées.

## Revendications

1. Dispositif de distribution de produit fluide, comportant un corps (1) pourvu d'un orifice de distribution (2), un réservoir (10) contenant au moins une dose de produit fluide étant disposé fixement dans ledit corps (1), ledit réservoir (10) étant obturé de manière étanche par deux bouchons (20, 30), un premier bouchon (20) et un second bouchon (30), le produit fluide étant disposé dans une chambre de produit (11) située entre lesdits premier et second bouchons (20, 30), ledit dispositif comportant des moyens d'ouverture (40) dudit premier bouchon (20), lesdits moyens d'ouverture (40) étant disposés fixement dans ledit corps (1), des moyens d'actionnement (50) étant reliés audit second bouchon (30) pour le déplacer dans ledit réservoir (10), l'actionnement dudit second bouchon (30) créant une pression dans la chambre de produit (11), ladite pression déplaçant et/ou déformant ledit premier bouchon (20) vers lesdits moyens d'ouverture (40), de telle sorte que ledit premier bouchon (20) est d'abord ouvert, puis du produit est expulsé à travers ledit premier bouchon (20) ouvert vers ledit orifice de distribution (2), **caractérisé en ce que** un profil de pulvérisation (5) est prévu en aval dudit orifice de distribution (2), le dispositif comportant des moyens de précompression (70) pour créer une précompression dans le réservoir (10) avant l'ouverture dudit premier bouchon (20) par lesdits moyens d'ouverture (40), pour améliorer la pulvérisation du produit et assurer la distribution de la totalité de la dose de produit.

2. Dispositif selon la revendication 1, dans lequel lesdits moyens d'ouverture (40) du premier bouchon (20) comportent une aiguille creuse (40) adaptée à percer ledit premier bouchon (20) et à transférer le produit fluide vers l'orifice de distribution (2).

3. Dispositif selon la revendication 1 ou 2, dans lequel ledit premier bouchon (20) est disposé à l'intérieur dudit réservoir (10) et est déplaçable vers lesdits moyens d'ouverture (40) lors de l'actionnement.

4. Dispositif selon la revendication 1 ou 2, dans lequel ledit premier bouchon (20) est fixé audit réservoir (10) par une partie fixe (21) et comporte une partie déformable (22), disposée à l'intérieur dudit réservoir (10), et se déformant vers lesdits moyens d'ouverture (40) lors de l'actionnement.

5. Dispositif selon la revendication 4, dans lequel ladite partie fixe (21) du premier bouchon (20) est fixée à l'extérieur du réservoir (10).

6. Dispositif selon l'une quelconque des revendications précédentes, dans lequel ledit réservoir (10) contient une dose unique de produit fluide distribuée lors d'un seul actionnement du dispositif.

7. Dispositif selon l'une quelconque des revendications 1 à 5, dans lequel ledit réservoir (10) contient au moins deux doses de produit fluide distribuées lors d'actionnements successifs des dispositifs, lesdits moyens d'actionnement (50) comportant des moyens de fractionnement de doses (60).

8. Dispositif selon l'une quelconque des revendications précédentes, dans lequel lesdits moyens de précompression (70) sont formés sur le premier bouchon (20) et/ou sur les moyens d'ouverture (40) et/ou sur le réservoir (10).

9. Dispositif selon la revendication 8, dans lequel lesdits moyens de précompression (70) comportent des moyens de résistance (71, 72, 73) nécessitant une force d'actionnement supérieure à un seuil prédéterminé pour ouvrir ledit premier bouchon (20).

10. Dispositif selon la revendication 9, dans lequel lesdits moyens de résistance (70) comprennent une butée coopérant avec ledit premier bouchon (20) avant son ouverture, ledit premier bouchon (20) devant être déformé pour surmonter ladite butée.

## Patentansprüche

1. Abgabevorrichtung für ein Fluidprodukt, aufweisend einen Körper (1), der mit einer Abgabeöffnung (2) versehen ist, einen Vorratsbehälter (10), der zumindest eine Fluidproduktdosis enthält, die in dem Körper (1) stationär angeordnet ist, wobei der Vorratsbehälter (10) durch zwei Stopfen (20, 30), einen ersten Stopfen (20) und einen zweiten Stopfen (30), dicht verschlossen ist, wobei das Fluidprodukt in einer Produktkammer (11) angeordnet ist, die zwischen dem ersten und zweiten Stopfen (20, 30) zu liegen kommt, wobei die Vorrichtung Öffnungsmittel (40) für den ersten Stopfen (20) umfasst, wobei die Öffnungsmittel (40) in dem Körper (1) stationär angeordnet sind, Betätigungsmittel (50), die mit dem zweiten Stopfen (30) verbunden sind, um diesen im Vorratsbehälter (10) zu verschieben, wobei die Betätigung des zweiten Stopfens (30) einen Druck in der Produktkammer (11) erzeugt; wobei der Druck den ersten Stopfen (20) in Richtung auf die Öffnungsmittel (40) verschiebt und/oder verformt, so dass der erste Stopfen (20) zunächst offen ist, bis Produkt durch den ersten Stopfen (20) ausgestoßen wird, der in Richtung auf die Abgabeöffnung (2) ausmündet, **dadurch gekennzeichnet, dass** ein Zerstäubungsprofil (5) stromabwärts von der Abgabeöffnung (2) vorgesehen ist, wobei die Vorrichtung Vorkompressionsmittel (70) umfasst, um eine Vorkompression in dem Vorratsbehälter (10) vor der Öffnung des ersten Stopfens (20) durch die Öffnungsmittel (40) zu erzeugen, um die Zerstäubung des Produkts zu verbessern und die Abgabe der gesamten Produktdosis zu gewährleisten.

2. Vorrichtung nach Anspruch 1, wobei die Öffnungsmittel (40) des ersten Stopfens (20) eine hohle Nadel (40) umfassen, die dazu ausgelegt ist, den ersten Stopfen (20) zu durchstechen und das Fluidprodukt in Richtung auf die Abgabeöffnung (2) zu übertragen.

3. Vorrichtung nach Anspruch 1 oder 2, wobei der erste Stopfen (20) im Innern des Vorratsbehälters (10) angeordnet und in Richtung auf die Öffnungsmittel (40) bei der Betätigung verschiebbar ist.

4. Vorrichtung nach Anspruch 1 oder 2, wobei der erste Stopfen (20) an dem Vorratsbehälter (10) durch einen feststehenden Teil (21) fest angebracht ist und einen verformbaren Teil (22) umfasst, der im Innern des Vorratsbehälters (10) angeordnet ist und sich in Richtung auf die Öffnungsmittel (40) bei der Betätigung verformt.

5. Vorrichtung nach Anspruch 4, wobei der feststehende Teil (21) des ersten Stopfens (20) an der Außenseite des Vorratsbehälters (10) festgelegt ist.

6. Vorrichtung nach einem der vorangehenden Ansprüche, wobei der Vorratsbehälter (10) eine einzige Fluidproduktdosis enthält, die bei einer einzigen Betätigung der Vorrichtung abgegeben wird.

7. Vorrichtung nach einem der Ansprüche 1 bis 5, wobei der Vorratsbehälter (10) zumindest zwei Fluidproduktdosen enthält, die bei aufeinander folgenden Betätigungen der Vorrichtung abgegeben werden, wobei die Betätigungsmittel (50) Dosisunterteilungsmittel (60) umfassen.

8. Vorrichtung nach einem der vorangehenden Ansprüche, wobei die Vorkompressionsmittel (70) auf den ersten Stopfen (20) und/oder auf den Öffnungsmitteln (40) und/oder auf dem Vorratsbehälter (10) gebildet sind.

9. Vorrichtung nach Anspruch 8, wobei die Vorkompressionsmittel (70) Widerstandsmittel (71, 72, 73) umfassen, die eine größere Betätigungskraft erfordern als eine vorbestimmte Schwelle, um den ersten Stopfen (20) zu öffnen.

10. Vorrichtung nach Anspruch 9, wobei die Widerstandsmittel (70) einen Anschlag umfassen, der mit dem ersten Stopfen (20) vor seiner Öffnung zusammenwirkt, wobei der erste Stopfen (20) verformt werden muss, um diese Schwelle zu übersteigen.

## Claims

1. A fluid dispenser device comprising a body (1) provided with a dispenser orifice (2), a reservoir (10) containing at least one dose of fluid being disposed statically in said body (1), said reservoir (10) being closed in sealed manner by two stoppers (20, 30), a first stopper (20) and a second stopper (30), the fluid being disposed in a fluid chamber (11) situated between said first and second stoppers (20, 30), said device further comprising opener means (40) for opening said first stopper (20), said opener means (40) being disposed statically in said body (1), actuator means (50) being connected to said second stopper (30) so as to displace it in said reservoir (10), the actuation of said second stopper (30) creating pressure in the fluid chamber (11); said pressure displacing and/or deforming said first stopper (20) towards said opener means (40), in such a manner that firstly said first stopper (20) is opened, and then fluid is expelled through said open first stopper (20) towards said dispenser orifice (2), the device being **characterized in that** a spray profile (50) is provided downstream from said dispenser orifice (2), the device further comprising precompression means (70) so as to create precompression in the reservoir (10) before said first stopper (20) is opened by said opener means (40), so as to improve the spraying of the fluid, and so as to ensure that the entire dose of fluid is dispensed.

2. A device according to claim 1, in which said opener means (40) for opening the first stopper (20) comprise a hollow needle (40) designed to pierce said first stopper (20) and to transfer the fluid towards the dispenser orifice (2).

3. A device according to claim 1 or claim 2, in which said first stopper (20) is disposed inside said reservoir (10) and is displaceable towards said opener means (40) during actuation.

4. A device according to claim 1 or claim 2, in which said first stopper (20) is fixed to said reservoir (10) by a fixed portion (21), and includes a deformable portion (22) that is disposed inside said reservoir (10), and that deforms towards said opener means (40) during actuation.

5. A device according to claim 4, in which said fixed portion (21) of the first stopper (20) is fixed on the outside of the reservoir (10).

6. A device according to any preceding claim, in which said reservoir (10) contains a single dose of fluid that is dispensed during a single actuation of the device.

7. A device according to any one of claims 1 to 5, in which said reservoir (10) contains at least two doses of fluid that are dispensed during successive actuations of the device, said actuator means (50) including dose-fractioning means (60).

8. A device according to any preceding claim, in which said precompression means (70) are formed on the first stopper (20), and/or on the opener means (40), and/or on the reservoir (10).

9. A device according to claim 8, in which said precompression means (70) comprise resistance means (71, 72, 73) requiring an actuating force that is greater than a predetermined threshold in order to open said first stopper (20).

10. A device according to claim 9, in which said resistance means (70) comprise an abutment co-operating with said first stopper (20) before it is opened, said first stopper (20) being deformable in order to be capable of moving past said abutment.
